# EUROPEAN PATENT SPECIFICATION

(11) **EP 2 726 203 B1**
(45) Date of publication and mention of the grant of the patent: **26.08.2020**
(21) Application number: 12745553.3
(22) Date of filing: 28.06.2012
(51) Int. Cl.: B01J 35/00, C04B 26/02, C04B 35/00

(54) **PHOTOCATALYTIC AND ANTI-BACTERIAL MATERIAL AND USE THEREOF IN THE BUILDING, LIGHTING AND FURNISHING FIELD**
PHOTOKATALYTISCHES UND ANTIBAKTERIELLES MATERIAL SOWIE DESSEN VERWENDUNG IM BAU-, BELEUCHTUNGS- UND EINRICHTUNGSBEREICH
MATÉRIAU PHOTOCATALYTIQUE ET ANTIBACTÉRIEN ET SON UTILISATION DANS LE DOMAINE DU BÂTIMENT, DE L'ÉCLAIRAGE ET DE L'AMEUBLEMENT

(30) Priority: 01.07.2011 IT MI20111233
(43) Date of publication of application: 07.05.2014
(73) Proprietor: Buzzi & Buzzi S.r.l., 20060 Pozzo d'Adda (Milano) (IT)
(72) Inventor: BUZZI, Dario, I-24040 Canonica d'Adda (Bergamo) (IT); BUZZI, Andrea, I-20069 Vaprio d'Adda (Milano) (IT); CHIARI, Maria Luisa, I-20060 Pozzo d'Adda (Milano) (IT)
(74) Representative: Villanova, Massimo
(86) International application number: PCT/IB2012/053271
(87) International publication number: WO 2013/005141

(56) References cited:
- EP-A1- 1 162 182
- WO-A1-2011/013797
- JP-A- 2007 083 146
- KR-A- 20060 024 747
- US-A1- 2010 201 022

## Description

The present invention regards an innovative material comprising a mixture of oxides and salts, aggregating agents, photocatalytic compounds and anti-bacterial compounds, and the use thereof for applications in the building, lighting and furnishing fields, for example for realizing supporting elements for lighting devices (lamps and other sources of light), room fittings, etc. As is known, in the construction of lamps and other devices for the illumination of environments, in general materials such as glass, metal (for example steel or aluminum), plastic, ceramic materials, plaster and cement are utilized. As regards recessed lamps in particular, the techniques currently in use have a series of drawbacks. For example, the use of plaster and/or cement does not offer satisfactory results in terms of mechanical performance, construction techniques and heat resistance. In addition, these are materials that are subject to hydraulic dehydration, which weakens them in relatively short periods of time (formation of cracks and tendency of the material to crumble). Another important defect lies in the difficulties involved in the use of such materials for realizing manufactured articles by means of injection-molding techniques, as it proves to be practically impossible to formulate these materials in such a manner as to obtain fluidity sufficient to fill the molds in which they are injected completely and homogeneously, resulting in the production of much rejected material and the need for touch-ups to be carried out on the final manufactured article.

As regards plastic materials, in general they do not allow for adequate thermal resistance, resulting in the possibility of permanent deformation of the manufactured article. Moreover, in the case of fire, many plastic materials can develop toxic fumes. Lastly, the paintability of these materials is generally poor, and in any case, not possible on the client's part after installation.

As regards glass materials, they are obviously fragile, with high thermal conductivity and cannot undergo pitch threading. Furthermore, they are markedly prone to surface accumulation of dust, smog and grease.

As regards metals, they are generally characterized by high thermal and electrical conductivity, they require a protective treatment of the surface and in addition they have high thermal expansion coefficients. These are factors that make their use unadvisable or even prevent their use in more extreme conditions (for example, outdoors or in very humid environments, particularly in high-salinity marine environments).

WO2011/013797 describes a photocatalytic material type of the glass-ceramic obtained from a composition comprising inter alia: tungsten oxide as a main component, calcium carbonate, zirconium oxide, zinc oxide, calcium carbonate, sodium nitrate, oxide silicon, aluminum oxide and silver.

JP2007083146 teaches the use of hydrogen-peroxide solution in the manufacture of photocatalytic foam ceramics.

Therefore, the demand remains strong in the sector for a material that does not suffer from the defects listed above, and that is characterized, among other things, by: very low thermal expansion and conductivity, high resistance to high temperatures, non-flammability, resistance to thermal shocks, high hardness, weather resistance (in particular to salinity in marine environments), dust repellency, mechanical resistance, abrasion resistance, absence of toxicity, absence of chemical reactivity, and non-allergenic characteristics. Moreover, said material must be versatile and possess high processability - above all by means of injection molding, a technique that makes it possible to realize manufactured articles with shapes that may even be complex and rich in details - and high productivity as well. Furthermore, the Applicant set herself the objective of combining all of these characteristics with the capacity to interact with the air in the environment in such a manner as to exert an effect of purification from polluting agents, as well as a bactericidal action. Therefore, an aim of the present invention is to provide an adequate response to the technical needs stated hereinabove.

The Applicant has unexpectedly found that a material comprising a mixture of oxides and salts, aggregating agents, photocatalytic compounds and anti-bacterial compounds as defined herein below, is capable of providing an adequate response to such needs.

According to a first aspect, the present invention thus regards a photocatalytic and anti-bacterial material according to claim 1.

In a second aspect, the present invention regards a process according to claim 8 for preparing a material as defined hereinabove, which comprises:
dry bulk premixing: calcium oxide, powdered marble, calcium sulfate, glass balls, sodium nitrate, silicon oxide, calcium carbonate, zirconium oxide;
adding an aqueous solution of hydrogen peroxide (H₂O₂);
adding the components from (b) to (f);
mixing so as to obtain a homogeneous composition.

It should be noted that the dry bulk mixing of the various components advantageously makes it possible to obtain a material having properties distributed in a homogeneous manner on the surface and throughout the bulk of the material, so as to ensure stable mechanical, thermal, photocatalytic and anti-bacterial performance over time. Advantageously, the addition of hydrogen peroxide gives rise to the development of oxygen, which is distributed in the form of microbubbles that promote the homogeneous mixing of the various components, decreasing the density of the agitated mass. Furthermore, in this manner, the heavier, non-reactive materials (for example the powdered marble) are prevented from settling when the aqueous solution is added. These microbubbles are then eliminated during the subsequent processing stages, so that they cannot undermine the mechanical resistance of the final manufactured article in any manner.

As regards the sequence for adding the various components to the agitated mass, it is not important in terms of the end result, so that they can be added in any order, separately or mixed together.

According to another aspect, the present invention regards a manufactured article according to claim 11 that comprises a photocatalytic and anti-bacterial material as defined hereinabove. In particular, this manufactured article can be selected for example from among: supporting elements for lighting devices, room fittings, grids for air-conditioner, etc.

According to a further aspect, the present invention regards a process according to claim 13 for the production of a manufactured article as defined hereinabove, that comprises injection molding of a photocatalytic and anti-bacterial material as described hereinabove.

Further characteristics and advantages of the present invention will become clearer from the description that follows, as also from the appended claims.

In a particularly preferred embodiment, the base mixture has the following composition:
- from 1.5% to 4.0% by weight, of calcium hydroxide, expressed as equivalent amount of calcium oxide, a precursor of calcium hydroxide by reacting with water;
- from 25.0% to 35.0% by weight, of powdered marble;
- from 10.0% to 20.0% by weight, of calcium sulfate;
- from 4.0% to 7.0% by weight, of glass balls;
- from 4.0% to 7.0% by weight, of sodium nitrate;
- from 2.0% to 5.0% by weight, of silicon oxide;
- from 20.0% to 30.0% by weight, of calcium carbonate;
- from 15.0% to 25.0% by weight, of zirconium oxide;
the percentages by weight being expressed with respect to the total weight of the base mixture.

Preferably, the material in accordance with the present invention comprises from 3.0% to 7.0% by weight, still more preferably from 4.0% to 6.0% by weight, of aluminum oxide, the percentages by weight being expressed with respect to the total weight of the base mixture.

Preferably, the material in accordance with the present invention comprises from 1.0% to 5.0% by weight, more preferably from 2.0% to 4.0% by weight, of zinc oxide, the percentages by weight being expressed with respect to the total weight of the base mixture.

Preferably, the material in accordance with the present invention comprises from 12.0% to 18.0% by weight, of zinc peroxide, the percentages by weight being expressed with respect to the total weight of the base mixture.

As regards the silver in an ionic form, it is generally added to the material in accordance with the present invention as an aqueous solution of a water-soluble silver salt, particularly silver nitrate. The amount of silver present in the material according to the present invention is expressed as added volume (in ml) of an aqueous solution containing 1% by weight of AgNO₃ per kg of the base mixture. This amount is from 10 to 50 ml/ kg, preferably from 20 to 40 ml/kg.

As regards the polymeric binder, it is preferably selected from among: polyvinyl acetate, optionally at least partially hydrolyzed; polyvinyl alcohol; or mixtures thereof. Preferably, the polymeric binder is added in the form of an aqueous emulsion. Preferably, the aqueous emulsion contains from 30% to 60% by weight, more preferably from 40% to 55% by weight, of polymer with respect to the total weight of the emulsion.

Preferably, the material in accordance with the present invention comprises from 1% to 5% by weight, of the polymeric binder, the percentages by weight being expressed with respect to the total weight of the base mixture.

In a preferred embodiment, the material in accordance with the present invention further comprises glass fibers, which allow for further enhancement of mechanical performance, acting as binders of the material in such a manner as to prevent the formation of cracks (anti-cracking additive), improving the surface quality, resistance to shocks and wear resistance. The glass fibers preferably have an average length from 3 to 30 mm, more preferably from 5 to 20 mm. The glass fibers preferably have an average diameter from 5 to 25 µm, more preferably from 10 to 20 µm. Preferably, the material in accordance with the present invention comprises from 0.2% to 5,0% by weight, more preferably from 0.5% to 2.0% by weight, of glass fibers, the percentages by weight being expressed with respect to the total weight of the base mixture.

As regards the calcium oxide, it is added to the base mixture as a setting accelerator, in that it gives rise to a strongly exothermal reaction when mixed with water:

CaO + H₂O ---> Ca(OH)₂ + 1136 kJ/kg CaO

The heat developed decreases the setting time in that it accelerates the hardening of the material and in the meantime it reduces the initial setting time, that is to say, the interval of time between the mixing of the components with water and the moment in which the mixture proves to be partially hardened and thus no longer workable in any manner.

Preferably, the calcium oxide has a particle size greater than or equal to 200 mesh, more preferably greater than or equal to 300 mesh. It should be noted that the higher the mesh-size number, the smaller the average size of the particles.

Preferably, the powdered marble prevalently consists of white marble, a petrographically homogeneous natural aggregate substantially constituted by fragments of white microcrystalline and saccharoid limestones. Said powdered marble preferably has an average particle size ranging from 0.005 to 2 mm, more preferably from 0.01 to 1 mm. Preferably, said powdered marble has a hardness ranging from 2 to 5 Mohs, preferably of about 3 Mohs; a moisture absorption rate lower than or equal to 1.5%; and a Class F1 freeze-thaw resistance.

As regards the calcium sulfate, it principally has the function of an extender, whereas the calcium carbonate essentially has the function of an opacifier and whitener of the material. The sodium nitrate instead principally performs a bacteriostatic function. These salts are added in the form of powder having a particle size generally greater than or equal to 200 mesh, preferably greater than or equal to 300 mesh.

As regards the silicon oxide, it principally performs the function of increasing the thermal resistance of the final material. Preferably, the silicon oxide is added in the form of powder having an average particle size lower than or equal to 0.1 µm, more preferably lower than or equal to 0.05 µm.

As regards the zirconium oxide, it principally performs the function of a catalyst, in the presence of visible light (photocatalyst) and also in the dark, for the purpose of facilitating the elimination of polluting substances present in the air, bacteria, molds, etc. Preferably, the zirconium oxide is added in the form of powder having a particle size greater than or equal to 200 mesh, more preferably greater than or equal to 300 mesh.

As regards the aluminum oxide, it principally has the task of improving the mechanical resistance of the final material. Preferably, the aluminum oxide is added in the form of powder having an average particle size lower than or equal to 5 µm, more preferably lower than or equal to 1 µm.

The zinc oxide principally performs the function of a setting accelerator so as to obtain a surface that is mechanically more resistant, and it also has photocatalytic properties. The zinc peroxide instead principally has the function of an antiseptic. Preferably, the zinc oxide and zinc peroxide are added in the form of powders having an average particle size lower than or equal to 10 µm, more preferably from 1 to 6 µm.

As regards the glass balls, they have the function of increasing the hardness, the mechanical resistance and the surface properties of the material. Preferably, the glass balls have the following features:
average diameter from 0.01 mm to 1.0 mm, more preferably from 0.1 to 0.5 mm; pH = 9-12; bulk density from 350 to 500 Kg/dm³; thermal conductivity lower than 0.15 W/mK, more preferably lower than 0.10 W/mK.

As regards the solution of hydrogen peroxide, it preferably has a concentration from 3 to 5%, and is added in an amount from 250 ml/Kg to 400 ml/Kg of the base mixture (a), more preferably from 300 ml/Kg to 350 ml/Kg.

For the preparation of the material according to the present invention, a step of dry bulk premixing of the various components of the base mixture (a) is carried out.

The mixing can be carried out with known prior-art devices for periods of time sufficient to obtain a perfectly homogeneous mixture. For this purpose, preferably a planetary mixer can be used from 400 to 900 r/min, preferably from 500 to 700 r/min, for a time interval ranging from 2 to 11 minutes, more preferably from 4 to 9 minutes, still more preferably from 5 to 8 minutes (principally based on atmospheric conditions such as humidity and temperature). The mixing is generally carried out at ambient temperature, preferably between 15 °C and 30 °C.

As mentioned previously, said stage of dry premixing of the components of the base mixture is important in order to obtain a final product in which all the ingredients are distributed in a homogeneous manner within the product and on the surface. The order in which the various components of the base mixture are added is not mandatory.

After the said bulk premixing step, which is always carried out by agitation, the solution of hydrogen peroxide is added, along with all the remaining additional components of the material according to the invention. In this second step as well, the order in which they are added is not mandatory. By way of example, it is possible to add the solution of hydrogen peroxide followed by all the other additional components, or the hydrogen peroxide in a mixture with the emulsion of the polymeric binder and then the other components, or following any other order.

In this second step as well, the mixing can be carried out with known prior-art devices for periods of time sufficient to obtain a perfectly homogeneous mixture, particularly by means of the same planetary mixer mentioned above, under conditions similar to those indicated above.

The final fluid composition thus obtained can be processed in various ways. In particular, to obtain the desired manufactured article, the composition is preferably subjected to injection molding, preferably at low temperature and low pressure, utilizing suitably predisposed molds having the shape and dimensions intended for the final manufactured article one desires to realize.

The injection conditions substantially depend on the type of machinery utilized, for example a screw injector.

In any case, the pressure applied ranges preferably from 1.85 MPa to 2.40 MPa; more preferably, from about 1.95 MPa to 2.30 MPa. The molding is preferably implemented at ambient temperature (preferably at 15 °C to 30 °C).

Following injection, the composition is kept inside the mold for the time needed for it to harden (setting time). After a setting time of 3-8 hours, preferably 4-6 hours, the desired manufactured article is extracted from the mold. Subsequently, it is preferably dried slowly, for example in a dryer for about 4-6 days at a temperature ranging from 50 °C to 90 °C, more preferably from 60 °C to 80 °C.

The manufactured article thus obtained has proved to possess very high-level mechanical characteristics (for example, compressive strength ≥ 55 N/mm² and a resistance to cutting ≥ 300 N/mm²).

Among other advantageous characteristics, the manufactured article has proved to possess powerful anti-bacterial activity, for example against *Escherichia coli, Klebsiella pneumoniae* and *Staphylococcus aureus,* pathogenic agents of particularly serious diseases. It also has an excellent ability to degrade the polluting substances present in urban environments such as hexane for example, which derives from the use of hydrocarbons as automotive or heating fuels.

These characteristics have been checked and confirmed by the TCNA-Tile *Council of North America,* which, by application of ISO 27447 *(Test method for anti-bacterial activity of semiconducting photocatalytic materials),* has demonstrated that in all samples of the invention tested, no survival of any bacterial was found after 8 hours in both UV-irradiated and dark test conditions. The same institution has also demonstrated that on the basis of the results of the tests performed using the UNI 11259 method (*Determination of photocatalytic activity of hydraulic binders, Rhodamine B method*), the samples of the invention tested showed a marked ability to degrade organic pollutants.

Lastly, the same institution has further demonstrated that in closed chamber conditions, n-hexane gas was degraded by 17.4 ppm in the presence of the tested sample of the invention.

Therefore, the product of the present invention contributes to purifying the air and to making environments cleaner, healthier and more hygienic, reducing the harmful effects of the principal polluting agents and unexpectedly exerting its action even in the absence of light.

The manufactured articles, and particularly the lamps, in accordance with the present invention thus prove to be particularly indicated for installation in all public settings (hospitals, health facilities, hotels, establishments) or private settings where there is a strongly perceived need to create a healthy environment. Moreover, the material according to the present invention has also proved to possess the following advantageous characteristics:
(1) absence of expansion: there is no formation of cracks during hardening or during installation;
(2) thermal resistance: the melting point of 1440 °C allows for a wide range of possibilities for the installation of light sources;
(3) extra fine surface of a silky, velvety appearance: it proves to be pleasing to the eye and to the touch and has a high degree of reflection, i.e., ≥ 97%;
(4) very low thermal conductivity: it can be utilized even in accessible areas without the risk of burns;
(5) non-flammability: it prevents the formation of toxic fumes;
(6) weather resistance: owing to its microporous structure, it has a porosity to water ≤ 2%;
(7) resistance to salinity: it proves to be particularly useful for outdoor and indoor installations in marine areas;
(8) resistance to freezing: it is insensitive to exfoliation due to freezing and thawing;
(9) hardness: it is highly resistant to cutting;
(10) abrasion resistance: the dustability of the surface is ≤ 7 gr/m².
(11) dust repellency: it does not require maintenance or frequent cleaning;
(12) resistance to UV rays: proves to be inalterable over time;
(13) paintability: if desired, it can also be painted even after installation.

## Claims

1. A photocatalytic and anti-bacterial material comprising:
a) a base mixture comprising:
calcium hydroxide;
powdered marble;
calcium sulfate;
glass balls;
sodium nitrate;
silicon oxide;
calcium carbonate;
zirconium oxide;
b) aluminum oxide;
c) zinc oxide;
d) zinc peroxide;
e) silver in an ionic form;
f) a polymeric binder;
wherein the base mixture has the following composition:
- from 1.0% to 5.0% by weight, preferably from 1.5% to 4.0% by weight, of calcium hydroxide, expressed as equivalent amount of calcium oxide, a precursor of calcium hydroxide by reacting with water;
- from 25.0% to 35.0% by weight of powdered marble;
- from 10.0% to 20.0% by weight of calcium sulfate;
- from 2.0% to 10.0% by weight, preferably from 4.0% to 7.0% by weight, of glass balls;
- from 2.0% a 10.0% by weight, preferably from 4.0% to 7.0% by weight, of sodium nitrate;
- from 1.0% to 8.0% by weight, preferably from 2.0% to 5.0% by weight, of silicon oxide;
- from 20.0% to 30.0% by weight of calcium carbonate;
- from 15.0% to 25.0% by weight of zirconium oxide;
the percentages by weight being expressed with respect to the total weight of the base mixture;
wherein the material comprises:
from 2.0 to 10.0% by weight, preferably from 3.0% to 7.0% by weight, more preferably from 4.0% to 6.0% by weight, of aluminum oxide, the percentages by weight being expressed with respect to the total weight of the base mixture;
from 0.5 to 7.0% by weight, preferably from 1.0% to. 5.0% by weight, more preferably from 2.0% to 4.0% by weight, of zinc oxide, the percentages by weight being expressed with respect to the total weight of the base mixture;
from 10.0% to 20.0% by weight, preferably from 12.0% to 18.0% by weight, of zinc peroxide, the percentages by weight being expressed with respect to the total weight of the base mixture;
from 0.5% to 10% by weight, preferably from 1% to 5% by weight, of the polymeric binder, the percentages by weight being expressed with respect to the total weight of the base mixture;
wherein the silver in an ionic form is added as an aqueous solution of a water-soluble silver salt, particularly silver nitrate, the amount of silver being from 10 to 50 ml/kg, preferably from 20 to 40 ml/kg, expressed as added volume (ml) of an aqueous solution containing 1% by weight of AgN03 per kg of the base mixture.

2. The material according to claim 1, wherein the polymeric binder is selected from: polyvinyl acetate, optionally partially hydrolyzed; polyvinyl alcohol; or mixtures thereof.

3. The material according to any one of the preceding claims, wherein the polymeric binder is added in the form of an aqueous emulsion.

4. The material according to any one of the preceding claims, which further comprises glass fibers, having an average length from 3 to 30 mm, preferably from 5 to 20 mm.

5. The material according to claim 4, wherein the glass fibers are present in an amount from 0.2% to 5.0% by weight, preferably from 0.5% to 2.0% by weight, the percentages by weight being expressed with respect to the total weight of the base mixture.

6. The material according to any one of the preceding claims, wherein:
calcium oxide has a particle size greater than or equal to 200 mesh, preferably greater than or equal to 300 mesh;
the powdered marble has an average particle size from 0.005 to 2 mm, preferably from 0.01 to 1 mm;
calcium sulfate, calcium carbonate and sodium nitrate are added in the form of powders having a particle size greater than or equal to 200 mesh, preferably greater than or equal to 300 mesh;
silicon oxide is added in the form of powder having an average particle size lower than or equal to 0.1 µm, preferably lower than or equal to 0.05 µm;
zirconium oxide added in the form of powder having a particle size greater than or equal to 200 mesh, preferably greater than or equal to 300 mesh;
aluminum oxide is added in the form of powder having an average particle size lower than or equal to 5 µm, preferably lower than or equal to 1 µm;
zinc oxide and zinc peroxide are added in the form of powders having an average particle size lower than or equal to 10 µm, preferably from 1 to 6 µm.

7. The material according to any one of the preceding claims, wherein the glass balls have the following features:
average diameter from 0.01 mm to 1.0 mm, preferably from 0.1 to 0.5 mm; pH = 9-12; bulk density from 350 to 500 Kg/dm<3>; thermal conductivity lower than 0.15 W/mK, preferably lower than 0.10 W/mK.

8. A process for preparing a material according to any one of the preceding claims, which comprises:
dry bulk premixing: calcium oxide, powdered marble, calcium sulfate, glass balls, sodium nitrate, silicon oxide, calcium carbonate, zirconium oxide;
adding an aqueous solution of hydrogen peroxide (H₂O₂);
adding the components from (b) to (f);
mixing so as to obtain a homogeneous composition.

9. The process according to claim 8, wherein the solution of hydrogen peroxide has a concentration from 3 to 5%, and is added in an amount from 250 ml/Kg to 400 ml/Kg of the base mixture (a), preferably from 300 ml/Kg to 350 ml/Kg.

10. The process according to claim 8 or 9, wherein the step/s of premixing and/or mixing is/are carried out by means of a planetary mixer.

11. A manufactured article that comprises a photocatalytic and anti-bacterial material according to any one of claims from 1 to 7.

12. The manufactured article according to claim 11, selected from: supporting elements for lighting devices, room fittings, grids for air-conditioner.

13. A process for producing a manufactured article according to claim 11 or 12, which comprises injection molding of a photocatalytic and anti-bacterial material according to any one of claims from 1 to 7.

## Patentansprüche

1. Fotokatalytisches und antibakterielles Material, umfassend:
a) ein Basisgemisch, umfassend:
Kalziumhydroxid;
gepulverten Marmor;
Kalziumsulfat;
Glaskugeln;
Natriumnitrat;
Siliziumoxid;
Kalziumcarbonat;
Zirkonoxid;
b) Aluminiumoxid;
c) Zinkoxid;
d) Zinkperoxid;
e) Silber in einer Ionenform;
f) ein polymeres Bindemittel;
wobei das Basisgemisch die folgende Zusammensetzung aufweist:
- von 1,0 bis 5,0 Gew.-%, bevorzugt von 1,5 bis 4,0 Gew.-%, Kalziumhydroxid, als äquivalente Menge von Kalziumoxid ausgedrückt, ein Vorprodukt von Kalziumhydroxid durch Reaktion mit Wasser;
- von 25,0 bis 35,0 Gew.-% gepulverten Marmor;
- von 10 bis 20 Gew.-% Kalziumsulfat;
- von 2,0 bis 10,0 Gew.-%, bevorzugt von 4,0 bis 7,0 Gew.-% Glaskugeln;
- von 2,0 bis 10,0 Gew.-%, bevorzugt von 4,0 bis 7,0 Gew.-% Natriumnitrat;
- von 1,0 bis 8,0 Gew.-%, bevorzugt von 2,0 bis 5,0 Gew.-% Siliziumoxid;
- von 20 bis 30 Gew-% Kalziumcarbonat;
- von 15 bis 25 Gew-% Zirkonoxid;
wobei die Gewichtsprozent in Bezug auf das Gesamtgewicht des Basisgemisches ausgedrückt sind;
wobei das Material umfasst:
von 2,0 bis 10,0 Gew-%, bevorzugt von 3,0 bis 7,0 Gew-%, noch mehr bevorzugt von 4,0 bis 6,0 Gew-% Aluminiumoxid, wobei die Gewichtsprozent in Bezug auf das Gesamtgewicht des Basisgemisches ausgedrückt sind;
von 0,5 bis 7,0 Gew-%, bevorzugt von 1,0 bis 5,0 Gew-%, noch mehr bevorzugt von 2,0 bis 4,0 Gew-% Zinkoxid, wobei die Gewichtsprozent in Bezug auf das Gesamtgewicht des Basisgemisches ausgedrückt sind;
von 10,0 bis 20,0 Gew-%, bevorzugt von 12,0 bis 18,0 Gew-% Zinkperoxid, wobei die Gewichtsprozent in Bezug auf das Gesamtgewicht des Basisgemisches ausgedrückt sind;
von 0,5 bis 10 Gew-%, bevorzugt von 1 bis 5 Gew-% des polymeren Bindemittels, wobei die Gewichtsprozent in Bezug auf das Gesamtgewicht des Basisgemisches ausgedrückt sind;
wobei das Silber in einer Ionenform als eine wässrige Lösung eines wasserlöslichen Silbersalzes, insbesondere Silbernitrat, hinzugefügt wird, wobei die Menge von Silber von 10 bis 50 ml/kg ist, bevorzugt von 20 bis 40 ml/kg, als hinzugefügtes Volumen (ml) einer wässrigen Lösung ausgedrückt, die 1 Gew-% AgNO3 pro kg des Basisgemisches enthält.

2. Material nach Anspruch 1, wobei das polymere Bindemittel ausgewählt ist aus: Polyvinylacetat, optional teilweise hydrolysiert; Polyvinylalkohol; oder Mischungen davon.

3. Material nach einem der vorstehenden Ansprüche, wobei das polymere Bindemittel in der Form einer wässrigen Emulsion hinzugefügt ist.

4. Material nach einem der vorstehenden Ansprüche, das weiter Glasfasern umfasst, die eine Durchschnittslänge von 3 bis 30 mm aufweisen, bevorzugt von 5 bis 20 mm.

5. Material nach Anspruch 4, wobei die Glasfasern in einer Menge von 0,2 bis 5,0 Gew-% vorliegen, bevorzugt von 0,5 bis 2,0 Gew-%, wobei die Gewichtsprozent in Bezug auf das Gesamtgewicht des Basisgemisches ausgedrückt sind.

6. Material nach einem der vorstehenden Ansprüche, wobei:
Kalziumoxid eine Teilchengröße größer als oder gleich 200 Mesh aufweist, bevorzugt größer als oder gleich 300 Mesh;
der gepulverte Marmor eine Durchschnittsteilchengröße von 0,005 bis 2 mm aufweist, bevorzugt von 0,01 bis 1 mm;
Kalziumsulfat, Kalziumcarbonat und Natriumnitrat in der Form von Pulvern hinzugefügt sind, die eine Teilchengröße größer als oder gleich 200 Mesh aufweisen, bevorzugt größer als oder gleich 300 Mesh;
Siliziumoxid in der Form von Pulver hinzugefügt ist, das eine Durchschnittsteilchengröße kleiner als oder gleich 0,1 µm aufweist, bevorzugt kleiner als oder gleich 0,05 µm;
Zirkonoxid in der Form von Pulver hinzugefügt ist, das eine Teilchengröße größer als oder gleich 200 Mesh aufweist, bevorzugt größer als oder gleich 300 Mesh;
Aluminiumoxid in der Form von Pulver hinzugefügt ist, das eine Durchschnittsteilchengröße kleiner als oder gleich 5 µm aufweist, bevorzugt kleiner als oder gleich 1 µm;
Zinkoxid und Zinkperoxid in der Form von Pulvern hinzugefügt sind, die eine Durchschnittsteilchengröße kleiner als oder gleich 10 µm aufweisen, bevorzugt 1 bis 6 µm.

7. Material nach einem der vorstehenden Ansprüche, wobei die Glaskugeln die folgenden Merkmale aufweisen:
Durchschnittsdurchmesser von 0,01 mm bis 1,0 mm, bevorzugt von 0,1 bis 0,5 mm; pH-Wert = 9-12; Bulkdichte von 350 bis 500 Kg/dm<3>; Wärmeleitfähigkeit kleiner als 0,15 W/mK, bevorzugt kleiner als 0,10 W/mK.

8. Prozess zur Zubereitung eines Materials nach einem der vorstehenden Ansprüche, der umfasst:
trockenes Bulkvormischen: Kalziumoxid, gepulverter Marmor, Kalziumsulfat, Glaskugeln, Natriumnitrat, Siliziumoxid, Kalziumcarbonat, Zirkonoxid;
Hinzufügen einer wässrigen Lösung von Wasserstoffperoxid (H₂O₂);
Hinzufügen der Komponenten von (b) bis (f);
Mischen, um eine homogene Zusammensetzung zu erhalten.

9. Prozess nach Anspruch 8, wobei die Lösung von Wasserstoffperoxid eine Konzentration von 3 bis 5% aufweist und in einer Menge von 250 ml/Kg bis 400 ml/Kg des Basisgemisches (a) hinzugefügt ist, bevorzugt von 300 ml/Kg bis 350 ml/Kg.

10. Prozess nach Anspruch 8 oder 9, wobei der Schritt (die Schritte) zum Vormischen und/oder Mischen mittels eines Planetenmischers ausgeführt ist (sind).

11. Hergestellter Artikel, der ein fotokatalytisches und antibakterielles Material nach einem der Ansprüche 1 bis 7 umfasst.

12. Hergestellter Artikel nach Anspruch 11, ausgewählt aus: Stützelementen für Beleuchtungsvorrichtungen, Raumausstattungen, Gitter für Klimaanlagen.

13. Prozess zur Fertigung eines hergestellten Artikels nach Anspruch 11 oder 12, der Spritzgießen eines fotokatalytischen und antibakteriellen Materials gemäß einem der Ansprüche 1 bis 7 umfasst.

## Revendications

1. Matériau photocatalytique et antibactérien comprenant :
a) un mélange de base, comprenant :
de l'hydroxyde de calcium ;
du marbre en poudre ;
du sulfate de calcium ;
des billes de verre ;
du nitrate de sodium ;
de l'oxyde de silicium ;
du carbonate de calcium ;
de l'oxyde de zirconium ;
b) de l'oxyde d'aluminium ;
c) de l'oxyde de zinc ;
d) du peroxyde de zinc ;
e) de l'argent sous forme ionique ;
f) un liant polymère ;
dans lequel le mélange de base présente la composition suivante :
- de 1,0 % à 5,0 % en poids, de préférence de 1,5 % à 4,0 % en poids, d'hydroxyde de calcium, exprimé comme quantité équivalente d'oxyde de calcium, un précurseur d'hydroxyde de calcium en le faisant réagir avec de l'eau ;
- de 25,0 % à 35,0 % en poids de marbre en poudre ;
- de 10,0 % à 20,0 % en poids de sulfate de calcium ;
- de 2,0 % à 10,0 % en poids, de préférence de 4,0 % à 7,0% en poids, de billes de verre ;
- de 2,0 % à 10,0 % en poids, de préférence de 4,0 % à 7,0 % en poids, de nitrate de sodium ;
- de 1,0 % à 8,0 % en poids, de préférence de 2,0 % à 5,0 % en poids, d'oxyde de silicium ;
- de 20,0 % à 30,0 % en poids de carbonate de calcium ;
- de 15,0 % à 25,0 % en poids d'oxyde de zirconium ;
les pourcentages en poids étant exprimés par rapport au poids total du mélange de base ;
dans lequel le matériau comprend :
de 2,0 à 10,0 % en poids, de préférence de 3,0 % à 7,0 % en poids, plus préférentiellement de 4,0 % à 6,0 % en poids, d'oxyde d'aluminium, les pourcentages en poids étant exprimés par rapport au poids total du mélange de base ;
de 0,5 à 7,0 % en poids, de préférence de 1,0 % à 5,0 % en poids, plus préférentiellement de 2,0 % à 4,0 % en poids, d'oxyde de zinc, les pourcentages en poids étant exprimés par rapport au poids total du mélange de base ;
de 10,0 % à 20,0 % en poids, de préférence de 12,0 % à 18,0 % en poids, de peroxyde de zinc, les pourcentages en poids étant exprimés par rapport au poids total du mélange de base ;
de 0,5 % à 10 % en poids, de préférence de 1 % à 5 % en poids, du liant polymère, les pourcentages en poids étant exprimés par rapport au poids total du mélange de base ;
dans lequel l'argent sous forme ionique est ajouté en tant que solution aqueuse d'un sel d'argent soluble dans l'eau, particulièrement un nitrate d'argent, la quantité d'argent allant de 10 à 50 ml/kg, de préférence de 20 à 40 ml/kg, exprimée en tant que volume ajouté (ml) d'une solution aqueuse contenant 1 % en poids d'AgN03 par kg du mélange de base.

2. Matériau selon la revendication 1, dans lequel le liant polymère est sélectionné parmi : l'acétate de polyvinyle, facultativement partiellement hydrolysé ; l'alcool polyvinylique ; ou des mélanges de ceux-ci.

3. Matériau selon l'une quelconque des revendications précédentes, dans lequel le liant polymère est ajouté sous la forme d'une émulsion aqueuse.

4. Matériau selon l'une quelconque des revendications précédentes, qui comprend en outre des fibres de verre, présentant une longueur moyenne de 3 à 30 mm, de préférence de 5 à 20 mm.

5. Matériau selon la revendication 4, dans lequel les fibres de verre sont présentes en une quantité de 0,2 % à 5,0 % en poids, de préférence de 0,5 % à 2,0 % en poids, les pourcentages en poids étant exprimés par rapport au poids total du mélange de base.

6. Matériau selon l'une quelconque des revendications précédentes, dans lequel :
l'oxyde de calcium présente une taille particulaire supérieure ou égale à 200 mesh, de préférence supérieure ou égale à 300 mesh ;
le marbre en poudre présente une taille particulaire moyenne de 0,005 à 2 mm, de préférence de 0,01 à 1 mm ;
le sulfate de calcium, le carbonate de calcium et le nitrate de sodium sont ajoutés sous la forme de poudres présentant une taille particulaire supérieure ou égale à 200 mesh, de préférence supérieure ou égale à 300 mesh ;
l'oxyde de silicium est ajouté sous la forme de poudre présentant une taille particulaire moyenne inférieure ou égale à 0,1 µm, de préférence inférieure ou égale à 0,05 µm ;
l'oxyde de zirconium ajouté sous la forme de poudre présentant une taille particulaire supérieure ou égale à 200 mesh, de préférence supérieure ou égale à 300 mesh ;
l'oxyde d'aluminium est ajouté sous la forme de poudre présentant une taille particulaire moyenne inférieure ou égale à 5 µm, de préférence inférieure ou égale à 1 µm ;
l'oxyde de zinc et le peroxyde de zinc sont ajoutés sous la forme de poudres présentant une taille particulaire moyenne inférieure ou égale à 10 µm, de préférence de 1 à 6 µm.

7. Matériau selon l'une quelconque des revendications précédentes, dans lequel les billes de verre présentent les caractéristiques suivantes :
un diamètre moyen de 0,01 mm à 1,0 mm, de préférence de 0,1 à 0,5 mm ; un pH = de 9 à 12 ; une masse volumique apparente de 350 à 500 kg/dm³ ; une conductivité thermique inférieure à 0,15 W/mK, de préférence inférieure à 0,10 W/mK.

8. Processus de préparation d'un matériau selon l'une quelconque des revendications précédentes, qui comprend les étapes consistant à :
prémélanger en vrac sec : l'oxyde de calcium, le marbre en poudre, le sulfate de calcium, les billes de verre, le nitrate de sodium, l'oxyde de silicium, le carbonate de calcium, l'oxyde de zirconium ;
ajouter une solution aqueuse de peroxyde d'hydrogène (H₂O₂) ;
ajouter les composants de (b) à (f) ;
mélanger de manière à obtenir une composition homogène.

9. Processus selon la revendication 8, dans lequel la solution de peroxyde d'hydrogène présente une concentration de 3 à 5 %, et est ajoutée en une quantité de 250 ml/kg à 400 ml/kg du mélange de base (a), de préférence de 300 ml/kg à 350 ml/kg.

10. Processus selon la revendication 8 ou 9, dans lequel l'étape/les étapes consistant à prémélanger et/ou mélanger est/sont effectuée(s) au moyen d'un batteur-mélangeur.

11. Article manufacturé qui comprend un matériau photocatalytique et antibactérien selon l'une quelconque des revendications 1 à 7.

12. Article manufacturé selon la revendication 11, sélectionné parmi : des éléments de support pour des dispositifs d'éclairage, des éléments d'agencement de pièce, des grilles pour climatiseur.

13. Processus de production d'un article manufacturé selon la revendication 11 ou 12, qui comprend le moulage par injection d'un matériau photocatalytique et antibactérien selon l'une quelconque des revendications 1 à 7.
